# EUROPÄISCHE PATENTSCHRIFT

(11) **EP 1 671 988 B1**
(45) Veröffentlichungstag und Bekanntmachung des Hinweises auf die Patenterteilung: **10.03.2010**
(21) Anmeldenummer: 05026192.4
(22) Anmeldetag: 01.12.2005
(51) Int. Cl.: C08G 18/02, C08G 18/79, C08G 18/10, C08G 18/48, C07C 267/00

(54) **Verfahren zur Herstellung flüssiger, lagerstabiler Carbodiimid- und/oder Uretonimingruppen aufweisender organischer Isocyanate mit niedriger Farbzahl**
Process for the preparation of liquid storage stable organic isocyanates with low colour index containing carbodiimide and/or uretonimino groups
Procédé pour la préparation d'isocyanates organiques liquides, stables au stockage, contenant des groupes carbodiimido et/ou uretonimino et ayant un indice de couleur faible

(30) Priorität: 14.12.2004 DE 102004060038
(43) Veröffentlichungstag der Anmeldung: 21.06.2006
(73) Patentinhaber: Bayer MaterialScience AG, 51368 Leverkusen (DE)
(72) Erfinder: Wershofen, Stefan, Dr., 41065 Mönchengladbach (DE); Schmidt, Manfred, Dr., 41450 Dormagen (DE); Pirkl, Hans-Georg, Dr., 51377 Leverkusen (DE); Hagen, Torsten, Dr., 45257 Essen (DE)

(56) Entgegenhaltungen:
- EP-A- 0 515 933
- EP-A- 1 054 029
- DE-A1- 2 606 419

## Beschreibung

Die Erfindung betrifft ein Verfahren zur Herstellung flüssiger, lagerstabiler Carbodiimid- (CD) und/oder Uretonimin- (UI) Gruppen aufweisender Isocyanatmischungen mit niedriger Farbzahl, die nach diesem Verfahren erhältlichen Isocyanatmischungen und deren -Verwendung zur Herstellung von Abmischungen mit weiteren Isocyanaten bzw. zur Herstellung von Isocyanatgruppen enthaltenden Prepolymeren sowie von Polyurethankunststoffen, vorzugsweise Polyurethanschaumstoffen.

CD- und /oder UI-Gruppen aufweisende Isocyanatmischungen können in einfacher Weise mit den hoch wirksamen Katalysatoren aus der Phospholin-Reihe, insbesondere der Phospholinoxid-Reihe, nach den Verfahren gemäß US-A-2,853,473, US-A-6,120,699 und EP-A-515 933 hergestellt werden.

Die hohe katalytische Aktivität der Phospholinkatalysatoren, insbesondere der Phospholinoxidkatalysatoren, ist einerseits erwünscht, um die Carbodiimidisierungsreaktion unter schonenden Temperaturbedingungen anzustoßen, andererseits ist aber bis heute kein Verfahren bekannt, das eine wirksame Abstoppung der Phospholin-Katalyse bzw. der Phospholinoxid-Katalyse ohne Einschränkung gewährleistet. Die carbodiimidisierten Isocyanate neigen zur Nachreaktion, d.h. sie gasen infolge CO₂-Entwicklung aus. Dies führt dann besonders bei höheren Temperaturen zu einem Druckaufbau beispielsweise in den Lagerbehältern.

Es hat nicht an Versuchen gefehlt, eine wirksame Abstoppung der Phospholin-Katalyse zu finden. Derartige Stopper sind z.B. in den Patentschriften DE-A-25 37 685, EP-A-515 933, EP-A-609 698 und US-A-6,120,699 erwähnt und umfassen z.B. Säuren, Säurechloride, Chloroformiate, silylierte Säuren und Halogenide der Hauptgruppenelemente. Ein Abstoppen des Katalysators mit Säuren, die z.B. auch als Säurechloride vorliegen können, ist nicht ausreichend wirksam.

Aus der DE-A-26 06 419 ist die Herstellung von lagerbeständigen, flüssigen Carbodiimidgruppen aufweisenden Polyisocyanaten, die wenig gefärbt sind, bekannt. Als Abstoppungs- und Stabilisierungsmittel werden Säurechloride, aromatische Sulfonsäureester oder Dimethylsulfat eingesetzt.

Nach der Lehre der EP-A-515 933 werden mittels Phospholin-Katalyse hergestellte CD/UI-haltige Isocyanatmischungen mit mindestens der äquimolaren Menge, bevorzugt der 1-2fachen molaren Menge bezogen auf den eingesetzten Katalysator an z.B. Trimethylsilyltrifluormethansulfonat (TMST) abgestoppt. In der Praxis hat sich jedoch erwiesen, dass solchermaßen hergestellte CD/UI-enthaltende Isocyanate zur Herstellung von Prepoymeren, d.h. Umsetzungsprodukten von diesen CD/UI-enthaltenden Isocyanaten mit Polyolen, nur bedingt geeignet sind. Die entsprechend hergestellten Umsetzungsprodukte aus Polyolen und den CD/UI-modifizierten Isocyanaten neigen zum Ausgasen, was zu einem Druckaufbau in den Transportbehältern oder zum Schäumen bei der Handhabung derartiger Produkte führen kann.

Man kann dieses Problem dadurch umgehen, dass man die zum Abstoppen des Phospholin-Katalysators benutzte silylierte Säure analog der EP-A-515 933 in höheren molaren Äquivalenten (z.B. 5:1-10:1 bezogen auf den Katalysator) einsetzt. In der Praxis zeigt sich dann jedoch, dass die erhaltenen CD/UI modifizierten Isocyanate eine deutlich schlechtere Farbzahl aufweisen. Dies gilt dann auch für die hieraus hergestellten Prepolymere.

Dies gilt auch, wenn der Phospholinkatalysator mit Säuren vom Typ der Trifluormethansulfonsäure entsprechend der US-A-6,120,699 abgestoppt wird. Auch daraus hergestellte Prepolymere weisen eine erheblich erhöhte Farbzahl auf.

Aufgabe der vorliegenden Erfindung war es daher, ein Verfahren zur Herstellung flüssiger, lagerstabiler und heller Carbodiimid- und/oder Uretonimingruppen aufweisender Isocyanatmischungen zur Verfügung zu stellen, welches die angesprochenen Mängel nicht aufweist und zu flüssigen, lagerstabilen Isocyanatmischungen mit niedrigen Farbzahlen führt.

Die Erfindung betrifft ein Verfahren zur Herstellung Carbodiimid- und/oder Uretonimingruppen aufweisender organischer Isocyanate, bei dem ein oder mehrere organische Isocyanate mit einer Hazen-Farbzahl von ≤ 100 APHA, bevorzugt ≤ 50 APHA, mit Katalysatoren vom Phospholin-Typ teilweise carbodiimidisiert werden, und anschließend die Carbodiimidisierungsreaktion abgestoppt wird, dadurch gekennzeichnet, dass als Stopper ein Alkylierungsmittel nach Anspruch 1 eingesetzt wird.

Die Messung der Hazen-Farbzahl kann dabei gemäß DIN/EN/ISO 6271-2 (Entwurf September 2002) in Substanz gegen Wasser als Referenz bei einer Schichtdicke von 5 cm erfolgen. Als Messgerät kann z. B. ein Photometer Dr. Lange LICO 300 eingesetzt werden.

Alkylierungsmittel sind Substanzen der allgemeinen Formel R-X, bei denen R einen organischen Rest und X die sogenannte Abgangsgruppe bezeichnet. Im Falle starker Alkylierungsmittel stellt X eine gute Abgangsgruppe dar, d.h. einen Rest, der nur eine geringe Nucleophilie aufweist. Anders ausgedrückt stellt X eine gute Abgangsgruppe dar, wenn X von einer starken Säure HX abgeleitet ist und damit eine sehr geringe Basizität aufweist. Als Verbindungen mit guten Abgangsgruppen werden in "Jerry March, Advanced Organic Chemistry, 3rd Edition (1985), John Wiley & Sons, Seite 315" z.B. Trialkyloxoniumverbindungen, Trifluormethansulfonate und Dialkylsulfate genannt.

Die Erfindung betrifft auch die Carbodiimid- und/oder Uretonimingruppen aufweisenden organischen Isocyanate, die nach dem obengenannten Verfahren erhältlich sind. Diese Carbodiimid- und/oder Uretonimingruppen aufweisenden organischen Isocyanate sind bei Raumtemperatur und in Abhängigkeit vom CD- / UI-Gehalt und/oder vom eingesetzten Isocyanat bis hin zu tiefen Temperaturen (z.B. 0°C) flüssig.

Gegenstand der Erfindung ist auch die Verwendung der erfindungsgemäßen Carbodiimid- und/oder Uretonimingruppen aufweisenden organischen Isocyanate zur Herstellung von Abmischungen mit weiteren Isocyanaten bzw. zur Herstellung von Isocyanatgruppen enthaltenden Prepolymeren mit verbesserter Farbzahl.

Gegenstand der Erfindung ist schließlich auch die Verwendung der erfindungsgemäßen Carbodiamid- und/oder Uretonimingruppen aufweisenden organischen Isocyanate und der daraus hergestellten Isocyanat-Abmischungen und/oder Prepolymere mit verbesserter Farbzahl zur Herstellung von Polyurethankunststoffen.

Überraschend können gemäß dem erfindungsgemäßen Verfahren flüssige, lagerstabile Isocyanatmischungen mit niedrigen Farbzahlen erhalten werden, im Gegensatz zur Lehre aus DE-A-25 04 334, in der z.B. Desaktivierung des Katalysators durch Alkylierungsmittel als nicht ausreichend beschrieben wird.

Alkylierungsmittel sind ebenfalls in US-A-4,424,288 zur Desaktivierung des Katalysators beschrieben, wenn als Einsatzmaterial ein rohes Polyphenylpolymethylenpolyisocyanat, das durch Anilin-Formaldehyd-Kondensation und anschließende Phosgenierung hergestellt werden kann ("rohes MDI"), und das einen Gehalt an Diisocyanatodiphenylmethan-Isomeren von maximal 70 Gew.-% aufweist eingesetzt wird. Das Ausgangsmaterial weist jedoch bereits an sich eine dunkle Farbe auf, so dass mit diesem Prozess kein farbhelles Produkt erhalten werden kann.

Als Ausgangsmaterialien für das erfindungsgemäße Verfahren können beliebige organische Isocyanate mit einer Hazen-Farbzahl von ≤ 100 APHA, bevorzugt ≤ 50 APHA, eingesetzt werden. Vorzugsweise wird das erfindungsgemäße Verfahren jedoch zur Carbodiimidisierung von organischen Diisocyanaten verwendet, die in der Polyurethan-Chemie eingesetzt werden.

Geeignet sind insbesondere:
- Aromatische Diisocyanate wie 2,4- und/oder 2,6-Diisocyanatotoluol (TDI), 2,2'-, 2,4'- und/oder 4,4'-Diisocyanatodiphenylmethan (MDI) bzw. beliebige Gemische derartiger aromatischer Diisocyanate,
- Polyisocyanatgemische der Diphenylmethanreihe mit einem Gehalt an monomeren Diisocyanatodiphenylmethan-Isomeren von 80 bis 100 Gew.-% und 0 bis 20 Gew.-% an höher als difunktionellen Polyisocyanaten der Diphenylmethanreihe, wobei sich die Diisocyanatodiphenylmethan-Isomeren zu 0 bis 100 Gew.-% aus 4,4'-Diisocyanatodiphenylmethan, 100 bis 0 Gew.-% aus 2,4'-Diisocyanatodiphenylmethan und zu 0 bis 8 Gew.-% aus 2,2'-Diisocyanatodiphenylmethan zusammensetzen, wobei sich die genannten Prozentsätze zu 100 % ergänzen.

Als Ausgangsmaterialien bevorzugte organische Isocyanate sind insbesondere aromatische Diisocyanate wie 2,4- und/oder 2,6-Diisocyanatotoluol (TDI), 2,2'-, 2,4'- und/oder 4,4'-Diisocyanatodiphenylmethan (MDI) bzw. beliebige Gemische derartiger aromatischer Diisocyanate. Besonders bevorzugt sind 2,2'-, 2,4'- und/oder 4,4'-Diisocyanatodiphenylmethan (MDI) bzw. beliebige Gemische derartiger aromatischer Diisocyanate, wobei die Summe an 2,2'-, 2,4'- und/oder 4,4'-Diisocyanatodiphenylmethan im Ausgangsmaterial (organisches Isocyanat) mindestens 85 Gew.% beträgt, und wobei sich die Düsocyanatodiphenylmethan-Isomeren zu 0 bis 100 Gew.-% aus 4,4'-Diisocyanatodiphenylmethan, 100 bis 0 Gew.-% aus 2,4'-Diisocyanatodiphenylmethan und zu 0 bis 8 Gew.-% aus 2,2'-Diisocyanatodiphenylmethan zusammensetzen, wobei sich die genannten Prozentsätze zu 100 % ergänzen. Ganz besonders bevorzugt sind 2,2'-, 2,4'- und/oder 4,4'-Diisocyanatodiphenylmethan (MDI) bzw. beliebige Gemische aromatischer Diisocyanate, wobei die Summe an 2,2'-, 2,4'- und/oder 4,4'-Diisocyanatodiphenylmethan im Ausgangsmaterial (organisches Isocyanat) mindestens 90 Gew.-% beträgt, und wobei sich die Diisocyanatodiphenylmethan-Isomeren zu 0 bis 100 Gew.-% aus 4,4'-Diisocyanatodiphenylmethan, 100 bis 0 Gew.-% aus 2,4'-Diisocyanatodiphenylmethan und zu 0 bis 8 Gew.-% aus 2,2'-Diisocyanatodiphenylmethan zusammensetzen, wobei sich die genannten Prozentsätze zu 100 % ergänzen. Insbesondere ganz besonders bevorzugt sind 2,2'-, 2,4'- und/oder 4,4'-Diisocyanatodiphenylmethan (MDI) bzw. beliebige Gemische aromatischer Diisocyanate, wobei die Summe an 2,2'-, 2,4'- und/oder 4,4'-Diisocyanatodiphenylmethan im Ausgangsmaterial (organisches Isocyanat) mindestens 99 Gew.-% beträgt, und wobei sich die Diisocyanatodiphenylmethan-Isomeren zu 0 bis 100 Gew.-% aus 4,4'-Diisocyanatodiphenylmethan, 100 bis 0 Gew.-% aus 2,4'-Diisocyanatodiphenylmethan und zu 0 bis 8 Gew.-% aus 2,2'-Diisocyanatodiphenylmethan zusammensetzen, wobei sich die genannten Prozentsätze zu 100 % ergänzen.

Das erfindungsgemäße Verfahren wird in Gegenwart von Katalysatoren vom Phospholin-Typ durchgeführt. Die Katalysatoren vom Phospholin-Typ sind beispielsweise aus EP-A-515 933 und US-A-6,120,699 bekannt. Typische Beispiele dieser Katalysatoren sind beispielsweise die aus dem Stand der Technik bekannten Gemische der Phospholinoxide der Formel:

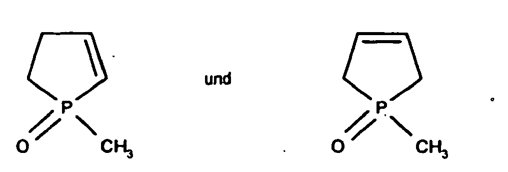

Die Menge des eingesetzten Katalysators hängt von der Qualität der Ausgangsisocyanate ab. Die jeweils notwendige Katalysatorenmenge lässt sich daher am einfachsten in einem Vorversuch bestimmen.

Die Carbodiimidisierungsreaktion wird üblicherweise im Temperaturbereich zwischen 50 bis 150°C, vorzugsweise von 60 bis 100°C, durchgeführt. Jedoch sind auch deutlich höhere Reaktionstemperaturen möglich (bis zu ca. 280°C). Die optimale Reaktionstemperatur richtet sich nach der Art der Ausgangsisocyanate und/oder des eingesetzten Katalysators und kann in einem einfachen Vorversuch ermittelt werden.

Die Carbodiimidisierungsreaktion wird im allgemeinen bei Erreichen eines Carbodiimidisierungsgrades (Carbodiimidisierungsgrad ist der Prozentsatz der carbodiimidisierten Isocyanatgruppen bezogen auf die Gesamtmenge der in Ausgangsisocyanat vorliegenden Isocyanatgruppen) von 3 bis 50 %, vorzugsweise 5 bis 30 %, abgebrochen.

Der Carbodiimidisierungsgrad kann während der Durchführung des erfindungsgemäßen Verfahrens durch Bestimmung des NCO-Wertes z.B. mittels dem Fachmann an sich bekannter Titration oder mittels online-Verfahren bestimmt werden. Ein geeignetes online-Verfahren ist z.B. die Nahinfrarot- oder die Mittelinfrarot-Analytik.

Der Carbodiimidisierungsgrad kann während der Durchführung des erfindungsgemäßen Verfahrens ebenfalls z.B. an der Menge des im Reaktorgemisch entweichenden Kohlendioxids erkannt werden. Diese volumetrisch bestimmbare Kohlendioxidmenge gibt somit zu jedem Zeitpunkt Auskunft über den erreichten Carbodiimidisierungsgrad.

Darüber hinaus können grundsätzlich auch andere geeignete, dem Fachmann bekannte offline- oder online-Methoden der Prozessverfolgung eingesetzt werden.

Zum Beenden der Carbodiimidisierungsreaktion wird als Abstopper bevorzugt mindestens die äquimolaren Menge, besonders bevorzugt der 1 - 20 fache molare Überschuss, ganz besonders bevorzugt der 1 - 10 fache molare Überschuss, bezogen auf den Katalysator, eines Alkylierungsmittels oder eines Gemisches verschiedener Alkylierungsmittel eingesetzt. Bevorzugt wird dabei das Alkylierungsmittel oder das Gemisch verschiedener Alkylierungsmittel als einziger Abstopper eingesetzt.

Vermutlich sind die Alkylierungsmittel in der Lage, die Katalysatoren vom Phospholinoxid-Typ zu alkylieren und auf diese Weise zu deaktivieren. Entsprechend sind erfindungsgemäß alle Alkylierungsmittel als Stopper geeignet, welche den Katalysator von Phospholinoxid-Typ durch Alkylierung zu deaktivieren und damit die Carbodiimidisierung abzustoppen vermögen. Geeignete Alkylierungsmittel sind für den Fachmann leicht durch einfache Versuche zu ermitteln.

Als Alkylierungsmittel werden Ester der Trifluormethansulfonsäure eingesetzt, die Verbindungen der Struktur CF₃-SO₃-R¹ sind, wobei R¹ einen (cyclo)aliphatischen oder araliphatischen Rest darstellt, der ggf. auch von Kohlenstoff und Wasserstoff verschiedene Atome enthalten kann. R¹ kann eine verzweigte oder lineare Kohlenstoffkette aufweisen und ggf. auch eine oder mehrere Kohlenstoff-Kohlenstoff-Mehrfachbindungen enthalten. Bevorzugt sind aliphatische Ester der Trifluormethansulfonsäure, besonders bevorzugt sind Methyl-, Ethyl-, Propyl-, Butyl- und Pentylester der Trifluormethansulfonsäure.

Alternativ zur alleinigen Verwendung von Alkylierungsmitteln als Abstopper kann in dem erfindungsgemäßen Verfahren eine Kombination eines Alkylierungsmittels mit einer Säure und/oder einem Säurechlorid und/oder einem Sulfonsäureester als zusätzlichem Stabilisator eingesetzt werden. Durch die Mitverwendung eines zusätzlichen Stabilisators kann die Einsatzmenge des Alkylierungsmittels reduziert werden. Die Zugabe dieses Stabilisators kann entweder zeitgleich mit der Zugabe des Abstoppers oder in einem nachgeschalteten Schritt erfolgen.

Als Säuren können ggf. halogenierte, aliphatische und/oder cycloalophatische und/oder aromatische Mono-, Di- und/oder Poly-Carbonsäuren wie z.B. Essigsäure, Adipinsäure, Cyclohexandicarbonsäure, α-Chlorpropionsäure, Benzoesäure, Phthalsäure, Isophtalsäure usw., sowie Sulfonsäuren, HCl, Schwefelsäure und/oder Phosphorsäure bzw. deren Mono- und/oder Diester, wie z.B. Dibutylphosphat, eingesetzt werden. Als Säurechloride können die aus den ggf. halogenierten, aliphatischen und/oder cycloalophatischen und/oder aromatischen Mono-, Di- und/oder Poly-Carbonsäuren bzw. Sulfonsäuren abgeleiteten Säurechloride sowie Carbamidsäurechloride, wie z.B. n-Butylcarbamidsäurechlorid, eingesetzt werden. Als Sulfonsäureester können z.B. p-Toluolsulfonsäuremethylester, p-Toluolsulfonsäureethylester eingesetzt werden.

Die zusätzlichen Stabilisatoren werden dabei in Mengen von insgesamt zwischen 10 und 1000 ppm (1 ppm: 1 Gewichtsteil auf 1000000 Gewichtsteile), bevorzugt zwischen 10 und 500, besonders bevorzugt zwischen 50 und 250 ppm, bezogen auf die Carbodiimid- und/oder Uretonimingruppen aufweisenden organischen Isocyanate, zugesetzt.

Das Reaktionsprodukt der Carbodiimidisierung kann Farbstabilisatoren enthalten, wie sie üblicherweise Isocyanaten zugesetzt werden. Dabei ist der Zeitpunkt des Zusatzes nicht kritisch. Die Farbstabilisatoren können entweder dem als Ausgangmaterial verwendeten Isocyanat vor der Carbodiimidisierung zugesetzt werden, oder dem Reaktionsprodukt nach vollendeter Umsetzung. Es ist ebenfalls möglich, Farbstabilisatoren sowohl dem Ausgangsmaterial als auch dem Reaktionsprodukt zuzugeben. Derartige Stabilisatoren sind generell dem Fachmann bekannt und umfassen z.B. Substanzen aus der Gruppe der sterisch gehinderten Phenole, der Phosphorigsäureester oder der tertiären Amine. Die Farbstabilisatoren können jeweils für sich allein oder im Gemisch mit anderen Vertretern der gleichen oder verschiedener Substanzgruppen eingesetzt werden. Die Mengen der eingesetzten Farbstabilisatoren bewegen sich in der dem Fachmann bekannten Größenordnung, üblicherweise im Bereich von 100 ppm bis 10000 ppm für die Einzelsubstanz bzw. das Gemisch, bezogen auf das als Ausgangsmaterial verwendete Isocyanat bzw. das Reaktionsprodukt der Carbodiimidisierung.

Es hat sich gezeigt, dass durch die Zugabe der Alkylierungsmittel allein oder in Kombination mit einer Säure und/oder einem Säurechlorid und/oder einem Sulfonsäureester als zusätzlichem Stabilisator nach dem erfindungsgemäßen Verfahren sowohl die Phospholin-Katalyse wirksam abgestoppt werden kann, als auch gleichzeitig niedrige Farbwerte im erzeugten Isocyanat und daraus hergestellten Prepolymeren erhalten werden können. Bei Einsatz der silylierten Säure als Abstopper gemäß dem Stand der Technik (EP-A-515 933) lässt sich die Phospholin-Katalyse nur durch Zugabe großer Mengen an silylierter Säure wirksam abstoppen, was aber zu erhöhten Farbwerten der so erzeugten Isocyanatmischungen und daraus hergestellten Prepolymeren führt.

Isocyanatgruppen enthaltende Prepolymere werden durch Umsetzung der nach dem erfindungsgemäßen Verfahren hergestellten Carbodiimid- und/oder Uretonimingruppen aufweisenden organischen Isocyanate mit in der Polyurethanchemie üblichen Polyolen erhalten. Geeignete Polyole sind sowohl einfache mehrwertige Alkohole des Molekulargewichtsbereiches 62 bis 599 g/mol, vorzugsweise 62 bis 300 g/mol, wie z.B. Ethylenglykol, Trimethylolpropan, Propandiol-1,2, Butandiol-1,2 oder Butandiol-2,3, Hexandiol, Octandiol, Dodecandiol und/oder Octadecandiol, insbesondere jedoch höhermolekulare Polyetherpolyole und/oder Polyesterpolyole der aus der Polyurethanchemie an sich bekannten Art mit Molekulargewichten von 600 bis 8000 g/mol, vorzugsweise 800 bis 4000 g/mol, die mindestens zwei, in der Regel 2 bis 8, vorzugsweise 2 bis 4 primäre und/oder sekundäre Hydroxylgruppen aufweisen. Beispiele derartiger Polyole sind in der US-PS 4 218543, Kolonne 7, Zeile 29 bis Kolonne 9, Zeile 32 beschrieben.

Die Vorteile des erfindungsgemäßen Verfahrens sind augenscheinlich: Sowohl die Carbodiimid und/oder Uretonimingruppen haltigen Isocyanate als auch die daraus hergestellten Prepolymere weisen eine gute Lagerstabilität und eine helle Farbe auf.

Diese Carbodiimid- und/oder Uretonimingruppen aufweisenden organischen Isocyanate und die daraus durch Umsetzung mit Polyolen hergestellten Prepolymere stellen wertvolle Ausgangsmaterialien zur Herstellung von Polyurethankunststoffen durch Umsetzung mit Polyolen (z.B. mit Polyetherpolyolen oder Polyesterpolyolen) nach dem Isocyanat-Polyadditionsverfahren dar.

### Beispiele

### Ausgangsprodukte:

- Desmodur 44M^{®}, Bayer AG (4,4'- Diphenylmethandiisocyanat, NCO-Gehalt: 33,6 Gew.-%)
- Katalysator vom Phospholinoxid-Typ: technisches Gemisch aus 1-Methyl-1-oxo-1-phosphacyclopent-2-en und 1-Methyl-1-oxo-1-phosphacyclopent-3-en, 10 %ig in Toluol
- Polyol: Multranol^{®} 3901, Bayer Corp. (Polyether aus Propylen- und Ethylenoxydeinheiten mit 80 - 90 % primären OH-Gruppen, einer Funktionalität von 3, einer OH-Zahl von 28 mg KOH/g und einer Viskosität von ca. 1200 mPas bei 25°C)

Allgemeine Vorschrift zur Herstellung des Carbodiimid- und/oder Uretonimingruppen aufweisenden organischen Isocyanates:
10 kg technisches 4,4'-MDI (Desmodur 44M^{®}) mit einer Hazen Farbzahl von < 15 APHA, das 750 ppm 3,5-Di-*tert*-butyl-4-hydroxytoluol enthält, werden unter N₂/Rühren auf 60°C erhitzt und mit 250 mg der Katalysatorlösung (2,5 ppm; 0,2 mmol) versetzt. Das Reaktionsgemisch wird unter N₂/Rühren für 240 min auf ca. 95°C erhitzt. Danach wird die Carbodiimidisierung durch Zugabe des jeweiligen Stoppers abgestoppt und 1 Stunde nachgerührt. In den erfindungsgemäßen Beispielen werden als Stopper Trifluormethansulfonsäuremethylester (TFMSME) bzw. Trifluormethansulfonsäureethylester (TFMSEE) und ggf. Isophthalsäuredichlorid (IPDC) in den in der Tabelle angegebenen Mengen eingesetzt. In den Vergleichsbeispielen 1 und 2 wird Trimethylsilyltrifluormethansulfonat (TMST) und in Vergleichbeispiel 3 Trifluormethansulfonsäure (TFMSS), jeweils in der in der Tabelle angegebenen Menge, verwendet. Die Ergebnisse sind in der nachfolgenden Tabelle zusammengefasst.

Allgemeine Vorschrift zur Herstellung eines Prepolymeren aus Carbodiimid- und/oder Uretonimingruppen aufweisendem organischen Isocyanat und einem Polyol:
Jeweils 500 g des nach vorstehender Vorschrift hergestellten Isocyanates werden bei 50°C unter N₂/Rühren mit 167 g Multranol^{®} 3901 versetzt und weitere 2 h bei 80°C unter N₂/Rühren gehalten. Die analytische Charakterisierung der Prepolymeren erfolgt am nächsten Tag. Zur Beurteilung der Stabilität der Prepolymere werden isotherme Druckversuche (12 h / 90°C) durchgeführt. Die Ergebnisse sind in der nachstehenden Tabelle zusammengefasst.

Die Messung der Hazen-Farbzahl erfolgt gemäß DIN/EN/ISO 6271-2 (Entwurf September 2002) in Substanz gegen Wasser als Referenz bei einer Schichtdicke von 5 cm. Als Messgerät kann z. B. ein Photometer Dr. Lange LICO 300 eingesetzt werden.

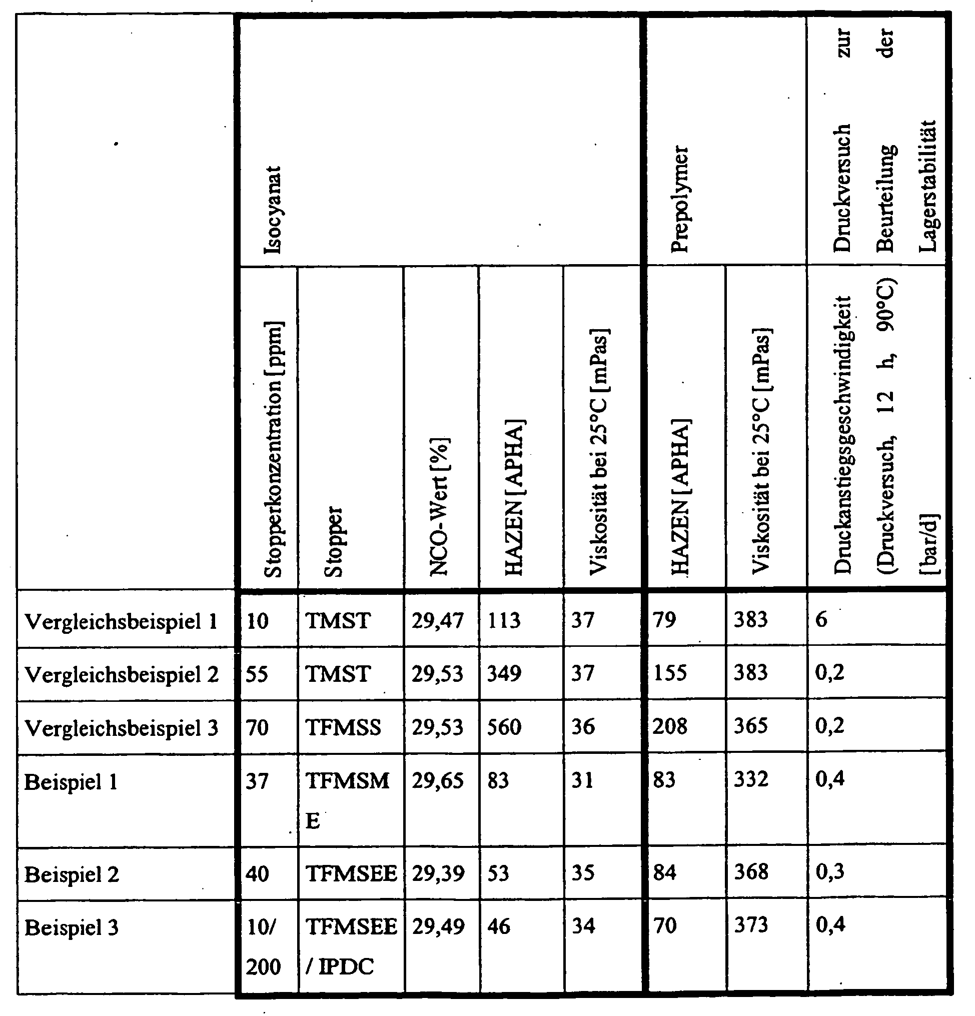

Vergleichsbeispiele 1 und 2 verdeutlichen den positiven Einfluss der erhöhten Stoppermenge an TMST auf die Stabilität, der jedoch zu Lasten der Farbe (HAZEN) geht. Vergleichsbeispiel 3 zeigt den nochmals ungünstigeren Einfluss des Stoppers Trifluormethansulfonsäure (TFMSS) auf die Farbe (HAZEN). In den erfindungsgemäßen Beispielen wird eine gegenüber Vergleichsbeispiel 1 verbesserte Stabilität erreicht und dabei das gute Farb-Niveau (HAZEN) erhalten.

## Patentansprüche

1. Verfahren zur Herstellung Carbodiimid- und/oder Uretonimingruppen aufweisender organischer Isocyanate, bei dem ein oder mehrere organische Isocyanate mit einer Hazen-Farbzahl von ≤ 100 APHA mit Katalysatoren vom Phospholin-Typ teilweise carbodiimidisiert werden, und anschließend die Carbodiimidisierungsreaktion abgestoppt wird, **dadurch gekennzeichnet, dass** als Stopper ein Alkylierungsmittel eingesetzt wird, wobei als Alkylierungsmittel die Ester der Trifluormethansulfonsäure der Struktur CF₃-SO₃-R¹ eingesetzt werden, wobei R¹ einen aliphatischen, cyclo-aliphatischen oder araliphatischen Rest darstellt.

2. Verfahren nach Anspruch 1, **dadurch gekennzeichnet, dass** als Stopper aliphatische Ester der Trifluormethansulfonsäure eingesetzt werden.

3. Verfahren nach einem der Ansprüche 1 bis 2, **dadurch gekennzeichnet, dass** als Stopper Methyl-, Ethyl-, Propyl-, Butyl- oder Pentylester der Trifluormethansulfonsäure eingesetzt werden.

4. Verfahren nach Anspruch 1, **dadurch gekennzeichnet, dass** zusätzlich zum Alkylierungsmittel eine Säure und/oder ein Säurechlorid und/oder ein Sulfonsäureester eingesetzt werden.

5. Carbodiimid- und/oder Uretonimingruppen aufweisende organische Isocyanate erhältlich nach dem Verfahren nach einem der Ansprüche 1 bis 4.

6. Verwendung der Carbodiimid- und/oder Uretonimingruppen aufweisenden organischen Isocyanate nach Anspruch 5 zur Herstellung von Isocyanat-Abmischungen.

7. Verwendung der Carbodiimid- und/oder Uretonimingruppen aufweisenden organischen Isocyanate nach Anspruch 5 zur Herstellung von Prepolymeren oder Polyurethanen.

## Claims

1. Process for the preparation of organic isocyanates containing carbodiimide and/or uretonimine groups, wherein one or more organic isocyanates with a Hazen colour index of ≤ 100 APHA are partially carbodiimidized with catalysts of the phospholine type, and the carbodiimidization reaction is then stopped, **characterized in that** an alkylating agent is used as the stopper, the alkylating agents used being trifluoromethanesulfonic acid esters of the structure CF₃-SO₃-R¹, in which R¹ is an aliphatic, cycloaliphatic or araliphatic radical.

2. Process according to Claim 1, **characterized in that** aliphatic trifluoromethanesulfonic acid esters are used as stoppers.

3. Process according to Claim 1 or 2, **characterized in that** methyl, ethyl, propyl, butyl or pentyl trifluoromethanesulfonate is used as the stopper.

4. Process according to Claim 1, **characterized in that** an acid and/or an acid chloride and/or a sulfonic acid ester are used in addition to the alkylating agent.

5. Organic isocyanates containing carbodiimide and/or uretonimine groups, obtainable by the process according to one of Claims 1 to 4.

6. Use of the organic isocyanates containing carbodiimide and/or uretonimine groups according to Claim 5 for the preparation of isocyanate mixtures.

7. Use of the organic isocyanates containing carbodiimide and/or uretonimine groups according to Claim 5 for the preparation of prepolymers or polyurethanes.

## Revendications

1. Procédé de préparation d'isocyanates organiques comportant des groupes carbodiimido et/ou urétonimino, dans lequel un ou plusieurs isocyanates organiques ayant un indice de coloration Hazen égal ou inférieur à 100 APHA sont partiellement carbodiimidés avec des catalyseurs de type phospholine, la réaction de carbodiimidation étant ensuite stoppée, **caractérisé en ce qu'**un agent alkylant est utilisé comme stoppeur, les esters de l'acide trifluorométhanesulfonique de la structure CF₃-SO₃-R¹, dans laquelle R¹ représente un radical aliphatique, cycloaliphatique ou araliphatique étant utilisés comme agent d'alkylation.

2. Procédé selon la revendication 1, **caractérisé en ce que** des esters aliphatiques de l'acide trifluorométhanesulfonique sont utilisés comme stoppeur.

3. Procédé selon l'une des revendications 1 à 2, **caractérisé en ce que** des esters méthyliques, éthyliques, propyliques, butyliques ou pentyliques de l'acide trifluorométhanesulfonique sont utilisés comme stoppeur.

4. Procédé selon la revendication 1, **caractérisé en ce qu'**un acide et/ou un chlorure d'acide et/ou un ester de l'acide sulfonique sont utilisés en plus de l'agent alkylant.

5. Isocyanates organiques présentant des groupes carbodiimido et/ou urétonimino, pouvant être obtenus selon le procédé selon l'une des revendications 1 à 4.

6. Utilisation des isocyanates organiques présentant des groupes carbodiimido et/ou urétonimino selon la revendication 5 pour la préparation de mélanges d'isocyanates.

7. Utilisation des isocyanates organiques présentant des groupes carbodiimido et/ou urétonimino selon la revendication 5 pour la préparation de prépolymères ou de polyuréthanes.
